# EUROPEAN PATENT APPLICATION

(11) **EP 2 749 264 A2**
(43) Date of publication of application: **02.07.2014**
(21) Application number: 11773985.4
(22) Date of filing: 31.08.2011
(51) Int. Cl.: A61K 8/25, A61K 8/29, A61K 8/37, A61K 8/39, A61K 8/92, A61K 8/97, A61Q 1/02, A61K 8/06, A61K 8/19

(54) **COSMETIC COMPOSITION INTENDED FOR MAKING UP THE SKIN, AND ARTICLE COMPRISING SAID COMPOSITION**

(71) Applicant: Natura Cosméticos S.A., 06882-700 ltapecerica da Serra - SP (BR)
(72) Inventor: ARATANI YANO, Bárbara, 05541-030 Sâo Paulo - SP (BR); DEL REY CASTRIOTTO, Alcione, 05709-020 Sâo Paulo - SP (BR); GANDINI ANDREO, Luciana, 05025-010 São Paulo -SP (BR)
(74) Representative: Pearson, Samuel John
(86) International application number: PCT/BR2011/000308
(87) International publication number: WO 2013/029127

(57) **Abstract**

The present invention relates to a cosmetic composition intended for skin makeup, being a water-in-oil emulsion comprising:
An oil phase comprising:
- an emollient system comprising at least one ester of plant origin;
- at least one optical diffuser;
- at least one pigment and

a water phase.

This cosmetic composition makes up the skin uniformly, presenting an integral covering of the skin region, good adhesion, minimizes excess gloss, provides smooth, satiny and velvety application to the skin, among other advantages desired in a product like this.

The present invention further relates to a cosmetic product comprising said cosmetic composition.

## Description

### Field of the Invention

The present invention relates to a cosmetic composition that is intended mainly for makeup of the skin. Further, the present invention relates to a cosmetic product comprising said cosmetic composition. Such a product is also preferably intended for makeup of the skin.

### Background of the Invention

Makeup is usually carried out by using colorful materials that are deposited onto the body or face.

The color is one of the main attributes of makeup. That is what makes it a cosmetic product for the purpose of essentially coloring.

From this basic conception, we have to consider that the ingredients responsible for producing color are of extreme importance, since they are responsible for the essential performance of these products.

Light is a physical agent capable of sensitizing our visual organs (retina). Light propagates by electromagnetic waves, that is, they can travel in vacuum (absence of matter).

As to its nature, it can be:
- Primary source or luminous bodies: those that emit light of their own; this is the case of the stars, the Sun, a candle flame, etc.;
- Secondary source or illuminated bodies: those that reflect the light which they receive to the space; this is the case of the moon, walls, clothes etc.

As to the emission:
- Simple or monochromatic source: the source of a single light, such as yellow light emitted by incandescent sodium vapor;
- Composed or polychromatic source: the source that results from superposition of lights of different colors; this is the case of white light emitted by the Sun and other sources.

Light reflection is one of the most important optical phenomena in our daily life; thanks to it we can see everything surrounding us.

The mechanism that describes this phenomenon is quite simple. A source of light emits light rays that fall onto the objects that surround it. After falling onto the objects, these rays return to the medium from where they came and thus they reach our eyes.

Depending on the object, reflected light arrives with greater or lesser intensity, since it may be more or less absorbed. Objects having a light surface reflect more light, while those having a dark surface have absorb more light.

Regular reflection takes place when, after falling onto a surface, a beam of light is reflected in a regular manner. This type of reflection takes place on mirrors, polished surfaces and water surface when the latter is not stirred, as for instance on the surface of a lake at a windless place.

Light diffusion takes place when light falls onto a rough surface, as is the case with most objects that surround us. When light falls onto these objects, it goes back to the medium of origin, becoming diffused, that is, it spreads. A very important consequence of this reflection is the fact that it enables us to see objects, people and the world around us.

Document US 2009081261 relates to a skin base including at least one dye having magnetic susceptibility different from zero, said base having a totality h located in the range from 30 to 70 degrees and lightness ranging from 25 to 80. The dye is selected in such a way that the base, under the action of a magnetic field, still can exhibit maximum variation of tonality lower than or equal to 5 DEG, and variation of luminosity higher than or equal to 4.

Document JP200434607 relates to a fluid-type makeup composition for the skin containing a liquid oil phase, a water phase and a copolyol dimeticone, and is in the form of a water-in-oil emulsion, in which there are solid polymethylmethacrylate particles, the liquid oily phase contains isododecane and the composition does not contain cyclotetrasiloxane.

Document FR 2812189 discloses a fluid base in the form of a water-in-oil emulsion comprising an oil phase and a water phase comprising polyol and optionally polymethyl methacrylate particles. The ratio between water and oil and polyol should be higher than or equal to 0.8.

Document WO98/26752 describes a base in the form of a water-in-oil emulsion, in which the water phase comprises: a) about 0.1% to about 10% of the base mass, of a water-soluble or water-dispersible polymer; b) about 0.5% to about 30% by mass of base, a plasticizing solvent and c) about 1% to about 30% by mass of base, a dispersion of ultra fine titanium dioxide in water. The dispersion of ultrafine titanium dioxide in water comprises about 0.2% to about 18% of the mass of the base of ultra fine titanium dioxide and about 0.002% to about 7.2% by mass of the base of a non-ionic surfactant.

In this regard, no prior-art document presents teachings relating to a liquid base with the characteristics and advantages listed hereinafter.

### Brief Description of the Invention

It is an objective of the present invention to provide a cosmetic composition intended for makeup of the skin (face and body), which is a water-in-oil emulsion that comprises:
(a) an oil phase comprising:
   - an emollient system comprising at least one ester of plant (vegetable) origin;
   - at least one optical diffuser; and
(b) an oil phase.

The present invention has also the objective of providing a cosmetic product comprising the above-described cosmetic composition.

### Detailed Description of the Invention

The present invention relates to a cosmetic composition intended for makeup of the skin (face and body), which is a water-in-oil emulsion comprising:
(a) an oil phase comprising:
   - an emollient system,
   - at least one optical diffuser;
   - at least one pigment; and
(b) a water phase.

Preferably, the cosmetic composition of the present invention is in the form of a water-in-oil emulsion and comprises an emollient system that has, in its oil phase, at least one ester of vegetable origin in an amount ranging from 0.5% to 5.0% by mass, at least one pigment in an amount ranging from 0.02% to 8.0% by mass and at least one optical diffuser in an amount ranging from 5% to 10% by mass, wherein the amounts are based on the total mass of the composition.

In a first preferred embodiment, the object of the present invention is in the form of a water-in-oil emulsion and comprises, in its oil phase, an emollient system comprising at least one ester of vegetable origin, which is a siliconized ester of sapucainha tree (*Carpotroche brasiliensis*)*,* a treated pigment that is a combination of iron oxide and titanium dioxide treated with ITT (Isopropyl Titanium Triisostearate) and an optical diffuser that is porous silica.

Further, the cosmetic composition of the present invention may be a cosmetic product like, for example, a liquid base for the face and neck, among other variations of liquid makeup for the skin.

The cosmetic product comprises the cosmetic composition described above in an amount ranging from 5.5% to 23% by mass, based on the total mass of the product.

The cosmetic composition of the present invention has a range of advantages and characteristics desired in a cosmetic product for the skin, these advantages being achieved with the optimum combination between the components already described, some of which are listed below:
- the cosmetic composition of the present invention presents a combination of technology and sustainability, improving the feeling and the performance of the product;
- some of the main components of the composition are obtained from a plant source;
- the cosmetic composition of the present invention provides uniform makeup of the skin, presenting an integral covering of the skin region where the composition has been applied, good adhesion and minimizes excessive gloss, resulting in a natural effect;

- the presence of an optical diffuser and treated pigments provides a natural finish to the skin and durability;
- the cosmetic composition of the present invention meets all the types of skin;
- the cosmetic composition of the present invention does not dry the skin and keeps the natural moisture thereof;
- optionally, the cosmetic composition of the present invention may comprise actives and other components that impart softness, velvety texture and revitalized look to the skin;
- the cosmetic composition of the present invention does not exhibit dermal irritation;
- the cosmetic composition of the present invention does not exhibit ocular irritation;
- the cosmetic composition of the present invention does not exhibit comedogenicity;
- the cosmetic composition of the present invention does not block the pores, allowing the skin to breath;
- the cosmetic composition of the present invention does not cause increase in oiliness of the skin, since it is free from oils;
- in preferred embodiments, it has ultrafine texture, achieving an even and natural result;
- the cosmetic composition of the present invention exhibits slide property, creaminess, softness and comfort that meet the desire of the consumer;
- it imparts luminosity and radiance to the skin;
- it imparts to the skin a soft-focus effect (minimizes lines/wrinkles/spots by a visual effect).

The main examples of cosmetic products that can be prepared by starting from the cosmetic composition of the present invention are:
- corrective;
- base;
- blush.

### Cosmetic Composition

As already said before, one claims herein a cosmetic composition intended for makeup of the skin (face and body), which is a water-in-oil emulsion comprising an oil phase comprising an emollient system containing at least one ester of plant origin, at least one optical diffuser, at least one pigment, and a water phase.

Preferably, the cosmetic composition of the present invention is in the form of a water-in-oil emulsion and comprises, in its oil phase, an emollient system comprising at least one ester of vegetable origin in an amount ranging from 0.5% to 5.0% by mass, at least one pigment in an amount ranging from 0.02% to 8.0% by mass and at least one optical diffuser in an amount ranging from 5.0% to 10.0% by mass, based on the total mass of the composition.

The cosmetic composition of the present invention has a combination of emollient system, treated pigment and light-diffusing particles that promote good feeling and unique slide. Further, in preferred embodiments, it constitutes a micronized product that imparts ultra fine texture and a silky feeling.

Further, by "feeling" one understands the sensation of contact of the cosmetic product with the skin. Thus, this concept embraces the refresh-ingness brought about when the product is applied to the skin, the emollience resulting in a velvety and silky touch, formation of a film over the skin, texture of the applied product, softness achieved, stickiness, among other attributes that result from the perception of the user when applying the product to the skin.

### Emollient System

The function of the emollient in cosmetic compositions is to add or replace natural oils of the skin, for the purpose of keeping the integrity of the hydrolipid coat of the skin. They can also act as sunscreen solubilizers. As emollient to be added to the composition of the present invention, one can use various substances of lipophilic nature and different polarities, such as alcohols and fatty acids, esters, ethers, mono-, di- or triglycerides, synthetic or natural hydrocarbons, or organic carbonates and combinations thereof.

Every emollient has an initial spreadability characteristic, spreadability-duration time and finally the residual after-drying feeling. In order to obtain desirable feeling characteristics, one opts for using specific emollients that provide a silky and velvety touch at the end of application of the product. If different emollients are combined, each of them can be felt on the growing order of emollience or decreasing order of time of spreadability duration.

In the cosmetic composition of the present invention the following emollients may be used, for example:
- 2-propylhepthyl caprylate: ester of plant origin, of light initial touch, high spreadability and quite fine and velvety final feeling. This ester is used at a concentration ranging from 1.0 to 10.0%, preferably 2.5 to 4.5%, more preferably 4.0%;
- Dicaprylic ether: ether of plant origin, of light initial touch, high spreadability and quite light final feeling. This ether is used at a concentration ranging from 0.5 to 7.0%, preferably 1.5 to 3.5%, more preferably 2.5%;
- Isopropyl palmitate: ester of plant origin that has medium spreadability and a more emollient initial feeling. At the end it leaves a little more creamy residue. Said ether is used at a concentration ranging from 0.5 to 6.0%, preferably from 1.0 to 3.0%, more preferably 2.0%;
- Jojoba ester; plant ester that has high spreadability and initially light and fine feeling, but that leaves a velvety and emollient film at the end. It provides matte final appearance (opaque effect, without excessive gloss). Said ester is used at a concentration ranging from 0.5% to 5.0%, preferably from 0.5% to 2.0%, more preferably 1.2%;
- Siliconized sapucainha tree ester: ester of plant origin, which has low spreadability and initially light and soft feeling, but that leaves a velvety and emollient film at the end. It provides matte final appearance. On a trained panel one can observe that the material can replace a totally synthetic raw material of high feeling performance. This ester is used at a concentration ranging from 0.5 to 5.0%, preferably from 0.5% to 2.0%, more preferably
1.2%.

Preferably, the emollient employed in the composition of the present invention is siliconized ester of sapucainha tree described above.

In preferred embodiments, one adds a combination of esters of plant origin that imparts spreadability properties to promote a light cosmetic composition, which provides a dry touch and is easy to apply, thus balancing the concentrations of existing solids.

Preferably, one adds to the emollient system a combination of esters of vegetable origin in an amount ranging from 3.0% to 33.0% by mass, preferably from 6.0% to 15.0% by mass, more preferably 10.75%, based on the total mass of the composition.

### Pigment

The pigment is responsible for the coloring achieved with the cosmetic composition of the present invention, being an essential component to achieve the technical advantages already described.

Pigments like titanium dioxide and iron dioxide are usually employed in makeup products. The fact is that, since these pigments have hydroxyl groups, they are potentially hydrophilic and absorb water on the molecule surface, which can form the agglomeration through the attraction forces of the hydrogen bridges.

Thus, a few important characteristics of the common pigments, that is, without treatment on their surface, are given below:
- the feeling is usually rougher and heavy due to the high friction between the agglomerates. Even when the pigments are micronized, there is a tendency to re-agglomeration;
- the pigments may migrate from the oil phase to the water phase in an emulsion, resulting in instability of the formulation;
- the surface of a few metal oxides may be highly reactive and cause degradation of other ingredients present in the formulation.

Preferably, the pigments are treated with a wide variety of compounds for modifying their physicochemical properties and preventing the above-cited points. The treatment technology promotes a very noticeable improvement on feeling, application, wettability of the pigments and stability of the formula.

Preferably, one uses in the cosmetic composition of the present invention the ITT (Isopropyl titanium triisostearate) treatment technology, capable of forming an even and lipophilic covering with the stearic groups present in the molecule and quite compatible with all the components present in the formulation, which is very relevant to promote a high final performance of the product.

The ITT coating is of plant origin, which enables greater affinity for the esters used in the formulations with the pigments, resulting in a greater stability of the formula and greater uniformity of the dispersion. Besides, the improvement in the feeling and in the manufacture process should also be pointed out.

Thus, this affinity too results in a cosmetic composition of optimum duration, since the fine film deposited onto the skin manages to sustain the color for a longer period of time, with high uniformity and ideal covering. It is similar to a color veil that melts with the microrelief of the skin, promoting a natural and lasting effect.

Further, the treated pigment enables one to achieve a high charge of particles for a good covering and aids in the optical diffusion property of the optical diffuser, which will be detailed later, without altering negatively its properties of application and fluidity. This is due to the fact that the ITT-coated pigment absorbs less carrier than a normal pigment (when the carrier is present), that is, the pigment remains protected and stable, but the color and the dispersion remains perfect and with high fluidity.

In a preferred embodiment, one uses, as a treated pigment, a combination of iron oxide and titanium dioxide, preferably treated with ITT, in an amount ranging from 0.01 % to 10.0% by mass, preferably from 3.5% to 7.0% by mass, more preferably 5%, based on the total mass of the composition.

### Optical Diffuser

The optical diffuser is a chemical component that diffuses, spreads or distributes light in some way, to make the illumination softer.

Preferably, one uses, as optical diffuser, porous silica.

More preferably, one uses silica (such as the product MSS500W from Kobo) of middle porosity in spherical format and with such a particle-size distribution that is capable of depositing in the valleys of the microrelief of the skin, promoting diffusion of the rays that fall onto the region, making the image slightly out of focus, resulting in the masking the lines and fine wrinkles present on the skin.

This product is further capable of promoting a very differentiated feeling, due to its composition and particle size, which, at the moment of application, causes each particle to be felt as slide and silky touch.

In a preferred embodiment, one adds at least one optical diffuser, porous silica being preferred, in an amount ranging from 5.0% to 10.0% by mass, preferably ranging from 7.0% to 9.0% by mass, more preferably 8.84%, based on the total mass of the composition.

Thus, this preferred embodiment presents ideal covering, capable of promoting uniformity with natural finish, besides masking fine lines with velvety finish through an optical diffuser.

### Optional Components

In order to impart the cosmetic composition of the present invention some desirable characteristic that has not yet been achieved with the components already cited, one may add optional components that are compatible with the properties thereof. A few of these components that may be added to the composition are those described later.

### Active

In a preferred embodiment, one may add anti-wrinkle actives to the cosmetic composition of the present invention. Preferably, one uses Elastinol+R, patented by the applicant itself. This is an oligosaccharide composition (sugars) rich in fucose and rhamnose, obtained through biotechnology.

Elastinol+R exhibits global action, acting on the main skin-aging mechanisms: elasticity, firmness, cell renewal, density and thickness.

Thus, Elastinol +R provides greater redensification and filling of the skin, besides treating the main skin-aging signs: wrinkles, slackness, loss of elasticity and thickness.

One may also add other actives to the cosmetic composition of the present invention.

In a preferred embodiment, one adds at least one active in an amount ranging from 0.5% to 3.0% by mass, based on the total mass of the composition.

### Antioxidant Complex

Antioxidants are capable of protecting the skin against free radicals, which are high reactive molecules, capable of interacting and destructing various types of important structures in the skin.

In a preferred embodiment, one adds the antioxidant complex already patented by the applicant and describes in summary hereinafter.

Coffee-beans extract - coffee-beans have, in their chemical composition, from 1 to 2.5% caffeine and various other substances that are present at higher concentrations, as for example chlorogenic acids.

Chlorogenic acids exhibit high antioxidant capability and are present in unripe coffee-beans at concentrations from 3 to 5 times as high as caffeine itself.

By an elaborate extraction process, the chlorogenic acids present in coffee beans are extracted and concentrated until one obtains a highly purified extract with excellent antioxidant capability for the skin.

On the skin, the anti-radical action of the Coffee-Beans Extract is proven through specific studies that demonstrate its capability of diminishing the oxidation of lipids (fats) of the membranes of the skin cells. Thus, the Coffee-Bean Extract aids in preserving important structures of the cell architecture.

Lycopene - this is a substance belonging to the chemical group of "carotenoids ", which are natural pigments synthesized by plants and some microorganisms. It belongs to the same family of beta-carotene, is extracted from tomato and imparts red color to the products.

Many studies have demonstrated its properties due to its special chemical structure. Lycopene is considered the caratenoid that has the greater anti-radical potential on one of the most important types of free radicals generated in the organism (there are different types and species of free radicals that act on our organism).

Vitamin E - It plays a fundamental role in preserving the cell membranes by its action of capturing free radicals formed ion the biochemical reactions of the skin or caused by exposure to sunshine. Thus, Vitamin E interrupts the cascade effect of forming more radicals, protecting the skin structures (colloidal gel, elastin and collagen) and minimizing the degradation thereof.

Studies carried out demonstrate that the antioxidant complex comprising lycopene, vitamin E, acetate and coffee-bean extract exhibit a synergistic effect, thus helping the natural defense system of the organism to fight, in a more effective manner, the free radicals formed from external aggressors like ultraviolet rays.

In embodiments of the invention, one adds at least one antioxidant in an amount ranging from 0.01 % to 0.5% by mass, based on the total mass of the composition.

### UVA and UVB Sunscreens

The sun is the greatest responsible for aging signs that appear on the skin. While UVB radiation is responsible for the formation of erythema, burns and spots, UVA radiation causes destruction of the sustaining fibers (collagen and elastin), leading to slackness and wrinkles.

Thus, the broad-spectrum sun protection (UVA and UVB) is fundamental in preventing photo-aging. In preferred embodiments, one adds to the cosmetic composition of the present invention a sunscreen system with photostable physical filters (titanium dioxide and zinc oxide), which impart SPF 15 (prevention of UVB) and also UVA-rays protection, guaranteeing efficiency and security, besides providing a pleasant feeling during and after application.

Preferably, the particles of physical filters undergo treatment to increase the stability of the particle, improving the feeling and efficacy of the UV protection. Preferably, one uses the combination of titanium dioxide and zinc oxide treated with ITT (Isopropyl titanium triisostearate). This treatment forms a uniform and lipophilic covering with the stearic groups present in the molecule, quite compatible with all the components present in the formulation, which is very relevant to promote a high final performance of the product.

The ITT coating is of plant origin, which enables a greater affinity for the ester used in the emollient system of the cosmetic composition of the present invention with the pigments, resulting in a greater stability of the formula and greater uniformity of the dispersion.

In addition to all these factors, there is further the great benefit of high affinity for the skin which promotes longer duration of the film formed to promote protection against the UV rays.

In embodiments of the invention, one adds at least one sunscreen in an amount ranging from 3.0% to 18.0% by mass, based on the total mass of the composition.

### Examples

The compositions exemplified above were tested, the results of the tests being detailed hereinafter.

### Example 1

### Patch Test: Potential of Irritability, Sensitization. Photoallergy and Cutaneous Phototoxicity - liquid Base

The awareness of the industry and the demands of the consumer have resulted in adoption of a new procedure on the part of the makers of cosmetics: at present, the companies are concerned with carrying out, prior to commercialization, clinical safety and efficacy tests, which are coordinated by dermatologist physicians. This procedure provides the company with greater security, credibility and reliability with the consumers.

A growing concern of the cosmetic industry is to prevent possible adverse reactions on the users of their products. After all, the consumer is much more critical with respect to the skin irritation caused by a cosmetic product than by a topical drug.

The Patch test is the main tool used in diagnosing reaction caused by cosmetic and in the allergenicity research. Since the main potential risks of using a new product are irritation, allergy by sensitization, phototoxicity and photoallergy, in the allergenicity research the following clinical tests are involved: primary and secondary skin irritability, skin sensitization, phototoxicity and photoallergy. These consist of repeated applications of the product to the skin and have the objective of detecting possible irritations or sensitization induction.

One can also evaluate, through these tests, in addition to allergenicity, the feeling characteristics of the product, detecting complaints and additional comments referring to its "performance".

Result: according to the methodology used to evaluate the potential of irritability, sensitization, photoallergy and skin phototoxicity of the product Liquid Base in question, one can conclude that the product did not induce irritation and skin sensitization process and did not cause photoallergy and phototoxicity during the period of study.

### Example 2

### Comedogenicity - Liquid Base

The volunteers selected were clinically evaluated by dermatologists in the beginning of the study (TO).

Then, a skilled professional carried out the counting of the number of comedones in a pre-delimited area by a mold 4.0cm wide by 4.5cm high, in the face area, with the aid of an illuminated magnifying class suitable for this purpose, as shown in the drawing below.
- Front Area: area of 4.0cm in width by 4.5cm in length, located medianly between the middle point of the globelle and the middle point of the front line by an imaginary line.
- Right or Left Half-Face Area: area of 4.0cm in width by 4.5cm in length, located medianly between the middle point of the lower eye-lid and the middle point of the menton line by an imaginary line.

After the evaluations, the samples were delivered to the volunteers according to guidance pertinent to the mode of use demonstrated hereinafter.

After 30 days of use, the volunteers returned for dermatological evaluation (T30), for counting of the comedones, carried out by a trained aesthetician. All the procedures described were written down on the clinical card and comedon count brochures of the volunteers.

Result: in the conditions in which the product was evaluated and in the sample of volunteers studied, the data enable one to conclude that no comedogenic potential was observed in the conditions of use, during the time of evaluation.

### Example 3

### Moisturizing - Liquid Base

In a monocentric study, one evaluated the product Liquid Base for its moisturizing efficacy on the skin of 20 volunteers.

Instruments evaluations were carried out for measuring the skin moisturizing by corneometry, the total duration of the test being of 01 week.

On each volunteer, one marked the areas treated and control on the forearms distributed at random. In the treated area with 40cm2, subdivided into 4 sub-areas of 10cm2, one applied the test product ion the proportion of 2mg/cm2. The control area did not receive any type of treatment.

Before each measurement the product was removed with 03 standardized cotton passes.

*Results:* the tested product exhibited moisturizing action, with a large amount of water present in the corneous layer of the population evaluated in all the experimental times, eliminating the control effect. This improvement was statistically significant: 15 minutes, 4 hours and 8 hours after the single application of the test product.

### Example 4

### Clinical Efficacy Test

The volunteers were evaluated by a dermatologist doctor through the Clinical Efficacy Questionnaire, in the following times:
- D0: on the first day of study, before using the product;
- D28: after 28± days of use of the product.

The following attributes were evaluated by dermatologist, in all the times cited, on the face skin of the volunteers: Skin Type, Wrinkle Degree, Amount of Wrinkle/Expression Lines, Intensity of the Wrinkles/Expression Lines, Aging Signs, Slackness Degree, Pocket Degree, Eye-Bags Degree, Eye-Bags, Firmness, Strength, Softness, Moisturizing, Drying, Texture, Oiliness and General Appearance.

### Evaluation / Criteria of Clinical Efficacy

The improvement of the skin evaluated by a dermatologist according to the attributes mentioned above. Visual and tactual evaluations were carried out.

### Evaluation of the noticed efficacy

The methodology used for evaluating the noticed efficacy was carried out according to *Standard Guide for Sensory Claim Substantiation.*

The volunteers were asked to evaluate the skin on the following time:
- D0: After 7+/-2 days from wash-out, prior to application of the product.

The attributes evaluated by the volunteers on D0 were: Skin Type, Softness, Moisturizing, Drying, Firmness, Elasticity, Density, Strength, Wrinkles/Expression Lines, Aging Signs, Oiliness , Pores, Vitality/Freshness, Texture, Silky Skin and General Appearance.

The volunteers received a mirror and were asked to evaluate the skin in the following times:
- D0-T10: after 7+/-2 days from wash-out, 10 minutes after the first application of the product;
- D7: after 7±2 days of use of the product;
- D14: after 14± days of use of the product;
- D28: after 28± days of use of the product.

The attributes evaluated by the volunteers on D0-T10 were: Correction Effect, Pores, Skin Gloss, Effect on Gloss, Texture, Uniformity of Ton, Natural Effect, Moisturizing, Silkiness, Vitality/Freshness and Formation of a Fine Protecting Film.

The attributes evaluated by the volunteers on D7, D14 and D28 were: Skin Type, State/present Skin Type, Change due to the product, Good Change, Product suitable for State/present Skin Type, Softness, Moisturizing, Drying, Firmness, Elasticity, Density, Strength, Wrinkles/Expression Lines, Aging Signs, Oiliness, Pores, Vitality/Freshness, Texture, Silky Skin and General Appearance.

The evaluation of noticed efficacy was conducted as a monadic, mono-blind testing.

The improvement of the skin was evaluated by the volunteers according to the attributes mentioned above, through visual and tactual evaluations.

Results: according to the methodology used for evaluating the product Liquid Base in question, one can conclude that:
After 28±2 days of use of the product (D28), the following statistically significant results were observed:
   - Improvement of the attributes Intensity of the Wrinkles/Expression Lines, Firmness, Elasticity, Strength, Softness, Moisturizing, Drying, Texture, Vitality/Freshness, Oiliness, Apparent Pores and General Appearance with respect to D0.

After 10 minutes from the first application of the product (D0-T10), the following results were observed:
- 45% of the volunteers considered the correction of the imperfections to be intense;
- 69% of the volunteers reported matte effect or half-matte effect;
- 83% of the volunteers considered the effect on the skin gloss to be good/excellent or good;
- 70% of the volunteers considered the skin tone to be very uniform or uniform;
- 95% of the volunteers considered that the skin got a natural effect;
- 92% of the volunteers noticed formation of a fine protecting film;
- 73% of the volunteers reported increase in the skin moisturizing;
- 73% of the volunteers considered that the pores become much

less apparent or became less apparent;
- 73% of the volunteers reported increase in the vitality/freshness of the skin;
- 67% of the volunteers reported improvement in the skin texture;
- 80% of the volunteers reported increase in the skin silkiness.

After 7+/- 2 days of use of the product (D7) the following results were observed:
- 77% of the volunteers considered that their state/skin type was different from the previous visit;
- 99% of the volunteers who reported change in the state/skin type considered that it was due to the use of the product;
- 100% of the volunteers who reported that the product provided change in the state of the skin considered the change to be good;
- 98% of the volunteers considered that the product was suitable for the state of their skin;
- 77% of the volunteers reported increase in skin softness;
- 70% of the volunteers reported increase in skin moisturizing;
- 73% of the volunteers reported reduction in skin drying;
- 47% of the volunteers reported increase in skin firmness;
- 36% of the volunteers reported increase in skin elasticity;
- 32% of the volunteers reported increase in skin density;
- 57% of the volunteers reported increase ion skin strength;
- 42% of the volunteers reported reduction of wrinkles/expression lines on the skin;
- 55% of the volunteers reported reduction of skin-aging signs;
- 58% of the volunteers reported reduction of skin oiliness;
- 57% of the volunteers considered that the pores became much less apparent or became less apparent;
- 65% of the volunteers reported increase in vitality/freshness of the skin;
- 84% of the volunteers reported improvement in skin texture;
- 75% of the volunteers reported increase in skin silkiness;
- 80% of the volunteers reported improvement in the general appearance of the skin.

After 14 ± 2 days of use of the product (D14), the following results were observed:
- 82% of the volunteers considered that their state/skin type was different from the previous visit;
- 100% of the volunteers that reported change in the state / skin type considered that it was due to the use of the product;
- 99% of the volunteers who reported that the product provided change in the state of the skin considered the change to be good;
- 99% of the volunteers considered that the product was suitable for the state of their skin;
- 84% of the volunteers reported increase ion skin softness;
- 80% of the volunteers reported increase in skin moisturizing;
- 79% of the volunteers reported reduction of skin drying;
- 63% of the volunteers reported increase ion skin firmness;
- 52% of the volunteers reported increase in skin elasticity;
- 54% of the volunteers reported increase in skin density;
- 72% of the volunteers reported increase in skin strength;
- 62% of the volunteers reported reduction of wrinkles/expression lines on the skin;
- 66% of the volunteers reported reduction of skin-aging signs;
- 71 % of the volunteers reported reduction in skin oiliness;
- 67% of the volunteers considered that the pores became much less apparent or became less apparent;
- 82% of the volunteers reported increase in vitality/freshness of the skin;
- 87% of the volunteers reported improvement in skin texture;
- 80% of the volunteers reported increase in skin silkiness;
- 93% of the volunteers reported improvement in the general appearance of the skin.

After 28 ± 2 days of use of the product (2d28), the following results were observed:
- 79% of the volunteers considered that their state/skin type was different from the previous visit;
- 99% of the volunteers who reported change in the state/skin type considered that it was due to the use of the product;
- 100% of the volunteers who reported that the product provided change in the state of the skin considered the change to be good;
- 100% of the volunteers considered that the product was suitable for the state of their skin;
- 89% of the volunteers reported increase in skin softness;
- 84% of the volunteers reported increase in skin moisturizing;
- 86% of the volunteers reported reduction in skin drying;
- 82% of the volunteers reported increase in skin firmness;
- 61 % of the volunteers reported increase in skin elasticity;
- 61 % of the volunteers reported increase in skin density;
- 84% of the volunteers reported increase in skin strength;
- 56% of the volunteers reported reduction of wrinkles/expression lines on the skin;
- 65% of the volunteers reported reduction of skin-aging signs;
- 71 % of the volunteers reported reduction of skin oiliness;
- 62% of the volunteers considered that the pores became much less apparent or less apparent;
- 82% of the volunteers reported increase in vitality/freshness of the skin;
- 93% of the volunteers reported improvement of skin texture;
- 88% of the volunteers reported increase in skin silkiness;
- 91% of the volunteers reported improvement of the general appearance of the skin.

## Claims

1. A cosmetic composition intended for skin makeup, being a water-in-oil emulsion, **characterized by** comprising:
(a) an oil phase comprising:
- an emollient system comprising at least one ester of plant origin;
- at least one optical diffuser;
- at least one pigment and
(b) a water phase.

2. A cosmetic composition according to claim 1, **characterized in that** the ester of plant origin is present in an amount ranging from 0.5% to 5.0% by mass, based on the total mass of the composition.

3. A composition according to any one of claims 1 and 2, **characterized in that** the ester of vegetable origin is selected from 2-propylheptyl caprylate, dicaprylic ether, isopropyl palmitate, jojoba ester, siliconized sapucainha-tree ester and combinations thereof.

4. A composition according to any one of claims 1 and 3, **characterized in that** the 2-propylheptyl caprylate is used at a concentration ranging from 0.5% to 6.0%, preferably 1.0% to 3.0%, more preferably 2.0%.

5. A composition according to any one of claims 1 and 3, **characterized in that** the dicaprylic ether is used at a concentration ranging from 0.5% to 7.0%, preferably 1.5% to 3.5%, more preferably 2.5%.

6. A composition according to any one of claims 1 and 3, **characterized in that** the isopropyl palmitate is used at a concentration ranging from 0.5% to 6.0%, preferably 1.0 to 3.0%, more preferably 2.0%.

7. A composition according to any one of claims 1 and 3, **characterized in that** the jojoba ester is used at a concentration ranging from 0.5 to 5.0%, preferably 0.5 to 2.0%, more preferably 1.2%.

8. A composition according to any one of claims 1 and 3, **characterized in that** siliconized sapucainha-tree ester is used at a concentration ringing from 0.5% to 5.0%, preferably 0.5% to 2.0%, more preferably 1.2%.

9. A composition according to any one of claims 1 to 3 and 8, **characterized in that** the ester of plant origin is preferably silizonized sapucainha-tree ester.

10. A composition according to claim 1, **characterized in that** the pigment is present in an amount ranging from 0.01 % to 10.0% by mass, based on the total mass of the composition.

11. A composition according to claim 1, **characterized in that** the pigment is a combination of iron oxide and titanium dioxide.

12. A composition according to claim 11, **characterized in that** the pigment is a combination of iron oxide and titanium dioxide treated with ITT.

13. A composition according to claim 1, **characterized in that** the optical diffuser is present in an amount ranging from 5% to 10% by mass, preferably ranging from 7.0% to 9.0% by mass, more preferably 8.84%, based on the total mass of the composition.

14. A composition according to any one of claims 1 to 5, **characterized in that** the diffuser is porous silica.

15. A cosmetic product **characterized by** comprising a cosmetic composition as defined in any one of claims 1 to 14.

16. A product according to claim 15, **characterized by** being a base.

17. A product according to claim 16, **characterized by** being a liquid base.

18. A product according to claim 15, **characterized by** being a corrective.

19. A product according to claim 15, **characterized by** being a blush.
